(19)

Europäisches
Patentamt
European
Patent Office
Office européen
des brevets

(11) **EP 3 533 475 A1**

(12) **EUROPEAN PATENT APPLICATION**

(43) Date of publication:
**04.09.2019 Bulletin 2019/36**

(51) Int Cl.:
***A61L 27/36*** (2006.01)  ***A61L 27/38*** (2006.01)

(21) Application number: **18159149.6**

(22) Date of filing: **28.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Centro Cardiologico Monzino SpA
20121 Milano (MI) (IT)**

(72) Inventors:
• **PESCE, Maurizio**
  **20121 Milano (IT)**
• **AMEDEO, Francesco**
  **20121 Milano (IT)**
• **SANTORO, Rosaria**
  **20121 Milano (IT)**

(74) Representative: **Trillat, Anne-Cecile et al
De Simone & Partners S.p.A.
Via Vincenzo Bellini, 20
00198 Roma (IT)**

(54) **METHOD FOR THE PRODUCTION OF PERICARDIAL MATERIAL**

(57)    The present invention relates to a method to produce a decellularized pericardial tissue or material comprising sequential incubation of a pericardial tissue with a non-ionic detergent and with a hypotonic detergent. Further, the invention relates to a decellularized pericardial tissue or material obtainable by the method and uses thereof. The invention also relates to an *ex vivo* method for producing recellularized pericardial tissue derived tissue comprising seeding of cells into the decellularized pericardial tissue and culture under oscillatory flow. The present invention further relates to the recellularized pericardial tissue obtainable by the *ex vivo* methods and to their medical uses in the field of heart valve engineering, particularly for heart valve implant, replacement, repair, or reconstruction.

**EP 3 533 475 A1**

## Description

### FIELD OF THE INVENTION

The present invention relates to a method to produce a decellularized pericardial tissue or material comprising sequential incubation of a pericardial tissue with a non-ionic detergent and with a hypotonic detergent. Further, the invention relates to a decellularized pericardial tissue or material obtainable by the method and uses thereof. The invention also relates to an *ex vivo* method for producing recellularized pericardial tissue derived tissue comprising seeding of cells into the decellularized pericardial tissue and culture under oscillatory flow. The present invention further relates to the recellularized pericardial tissue obtainable by the *ex vivo* methods and to their medical uses in the field of heart valve engineering, blood vessels, particularly for heart valve implant, replacement, repair, or reconstruction.

### BACKGROUND TO THE INVENTION

Animal-derived pericardium is the elective tissue employed in manufacturing heart valve prostheses. The preparation of this tissue for biological valves production consists in fixation with aldehydes, which reduces but not eliminates the xenoantigens and the donor cellular material. As a consequence, especially in patients below 65-70 years of age, the employment of pericardium containing valve substitutes is not indicated due to chronic rejection that causes tissue degeneration and recurrence of valve insufficiency. Decellularization with ionic/non-ionic detergents has been proposed as an alternative procedure to prepare aldehyde-/xenoantigen-free pericardium for biological valve manufacturing.

Calcific Aortic Valve Disease (CAVD) is one of the cardiac pathologies whose incidence is expected to grow more in the future, due to the worldwide elevation of life expectancy. The current procedures to treat CAVD consist in replacement with mechanical or biological substitutes (Schoen, 2008, Schoen and Gotlieb, 2016). Mechanical valves are manufactured with various materials, especially metal alloys, which provide the advantage to make them structurally and mechanically unmodifiable. Mechanical valve substitutes have, however, a major shortcoming in the need to treat patients with continuous anticoagulant administration to prevent the formation of thrombi (David, 2013). This problem makes these valves unsuitable in elderly patients, who may have a too high risk of bleeding (Head, et al., 2017). Bioprosthetic valves are manufactured with biological materials such as complete valves (from porcine) or animal-derived pericardium (from bovine or porcine) (Head, et al., 2017, Namiri, et al., 2017, Okamoto, et al., 2016, Yap, et al., 2013). The advantage of this type of valve replacements consists in the lack of need to constantly administer anticoagulation therapy. In addition, the adoption of minimally invasive procedures to implant biological valves (the so called Trans Aortic Valves Implants, TAVI) manufactured with animal pericardium, has greatly reduced the operative and post-operative complications, making these devices the preferred option for ederly patients with CAVD (Nishimura, et al., 2014, Vahanian, et al., 2012).

Irrespective whether pericardial-made prostheses are manufactured as conventionally or minimally invasively implantable devices (Chacko, et al., 2013, Farias, et al., 2012, Johnston, et al., 2015), they are 'xenografts' whose integration in the host fails at mid-long term for lack of a complete immunological compatibility and insufficient fixative detoxification. In fact, the treatment of the animal tissues with aldehyde-based fixatives leaves exogenous immunoreactive biological material (e.g. DNA), xenoantigens (e.g. the 1,3 $\alpha$-Galactose) and aldehyde residues which determine a chronic rejection, resulting into leaflet inflammation/calcification and progressive deterioration of mechanical performance (Carpentier, et al., 1969, Grabenwoger, et al., 1996, Schoen and Levy, 2005, Siddiqui, et al., 2009). Improvement of fixation methods and treatment with agents that detoxify aldehyde residues and xenoantigens have been proposed as strategies to improve the compatibility of the pericardium and reduce its calcification potential (Flameng, et al., 2014, Guldner, et al., 2009, Leong, et al., 2013, Lim, et al., 2012). Lastly, decellularization procedures to prepare xenoantigen-free acellular pericardium have been set, based on treatments of the tissue with hypotonic buffers and ionic/non-ionic detergents such as SDS and TritonX-100 (Korossis, et al., 2002, Mirsadraee, et al., 2006, Mirsadraee, et al., 2007). Although these methods have been proven to maintain a relatively good mechanical performance, to abolish the xenoantigen content (Boer, et al., 2012, Gardin, et al., 2015, Ghodsizad, et al., 2014, Roosens, et al., 2016, Wong, et al., 2013), and to reduce the tissue immunogenicity (Paola, et al., 2017, Wong, et al., 2016), thus far there has been a limited employment of these methods for preparation of valve replacement devices at an industrial scale (Horke, 2016).

In a previous investigation (Vinci, et al., 2013), the inventors proposed a modification of a standard decellularization method (Mirsadraee, et al., 2006, Mirsadraee, et al., 2007), to obtain a complete cell removal from the human pericardium. In this work, the immunological compatibility of the human tissue was shown by subcutaneous transplantation of the decellularized pericardium in immunocompetent mice. The decellularization method was also shown not to alter the three-dimensional structure and the composition of the extracellular matrix, as well as the tissue mechanical behavior. Santoro, et al., 2016 describes an *ex vivo* method of obtaining a decellularized pericardial tissue consisting in a sequential incubation of the pericardial tissue into a hypotonic buffer, a detergent hypotonic solution containing 0.1% (w/v) sodium dodecylsulfate (SDS) and a nucleic acid removal solution containing deoxyribonuclease.

When the method was tested to assess the compatibility of the animal-derived pericardium for tissue engineering applications (Santoro, et al., 2016a), the inventors found a complete removal of animal xenoantigens and the lack of toxic residuals that could impair growing of aortic valve-derived interstitial cells, the cell type naturally deputed to valve tissue homeostasis and matrix renewal (Liu, et al., 2007a, Taylor, et al., 2003).

There is still a need for a pericardium derived tissue for heart valve implant, replacement, repair, or reconstruction that is biologically compatible while not deteriorating structurally, failing mechanically, or leading to rupture of the prosthesis leaflet.

## BRIEF DESCRIPTION OF THE INVENTION

In the present invention, an original fixation-free decellularization method was set up, to obtain cell-free pig pericardium patches. After decellularization and testing, the tissue was seeded with cells derived from pig-derived aortic valves using a perfusion bioreactor. Results showed a complete and stable cellularization of the decellularized tissue, thus demonstrating the generation of a living pericardium-based tissue suitable to be employed as valve leaflets in off the shelf biovalve implants. The present decellularization procedure was optimized to allow seeding and culturing of valve competent cells able to colonize and reconstitute a valve-like tissue. A high efficiency cellularization was achieved by forcing cell penetration inside the pericardium matrix using a perfusion bioreactor. Since the decellularization procedure was found not to alter the collagen composition of the pericardial matrix and cells seeded in the tissue constructs consistently grew and acquired the phenotype of 'quiescent' valve interstitial cells (VICs), the present invention sets a novel standard in pericardium application for tissue engineering of 'living' valve implants. In the present invention, a direct perfusion bioreactor was employed to achieve an efficient and homogeneous delivery of valve interstitial cells into the decellularized pericardium (Cioffi, et al., 2008, Wendt, et al., 2003b). The results showed an optimal growth of valve-derived cells inside the tissue, and a partial maturation as a living leaflet tissue. Compared to less efficient (Dainese, et al., 2012) or technology demanding approaches proposed in the literature to engineer completely recellularized valves (Converse, et al., 2017, Ghodsizad, et al., 2014), the method of the present invention appears an affordable and easily scalable procedure that may be industrially implemented for generating living 'off-the-shelf valve replacement tissue.

The fixative-free decellularization method of the present invention presents several advantages as compared to previous methods, such as the reduction of immunogenic reactions and calcification, the maintenance of the mechanical properties, the increase in tissue permeability to allow proper recellularization, the decrease in lipid and increase in collagen contents.

Further, in vitro dynamic recellularization may enhance functional performance in the early stage of postoperation and in vitro dynamic recellularization adapts to body growth (pediatric patients) and may have less inflammatory response.

It is therefore an object of the invention an *ex vivo* method for producing decellularized pericardial material comprising:

a) incubation of a pericardial tissue in a hypotonic solution supplemented with at least one protease inhibitor;
b) incubation of the pericardial tissue incubated in a) in a 1% (v/v) Triton X-100 solution in bi-distilled water;
c) incubation of the pericardial tissue incubated in b) into a 0.1% (w/v) sodium dodecylsulphate (SDS) in bi-distilled water.

Preferably the hypotonic solution is 10 mM Tris-HCl having a pH 8.0. Preferably any of steps a), b) or c) is carried out for 1 to 24 hours at a temperature between 2 and 8 °C. Preferably any of steps a), b) or c) is carried out independently for 24 hours at a temperature of 4 °C.

Preferably said pericardial tissue is porcine, bovine, human, equine.

The present invention also provides a decellularized pericardial material obtainable by the *ex vivo* method as defined above.

Preferably the decellularized pericardial material is characterized in that it has a permeability lower than 1.0 x $10^{-13}$ $m^4$/Ns.

Preferably the decellularized pericardial material is characterized in that it has a thickness comprised between 100 $\mu$m and 1000 $\mu$m.

Preferably the decellularized pericardial material is characterized in that essentially all cellular proteins have been removed.

The present invention provides the use of the decellularized pericardial material as defined above for the production of living tissue incorporating cells.

The present invention also provides an *ex vivo* method for producing cellularized pericardial tissue comprising contacting the decellularized pericardial material as defined above with autologous or heterologous cells selected from the group consisting of: valve-derived interstitial cells, adipose-tissue stromal/mesenchymal cells, myocardial stromal/mesenchymal cells, Wharton Jelly stromal/mesenchymal cells and vascular-derived stromal/mesenchymal/progenitor cells or a mixture thereof, by means of an oscillatory flow, preferably in a bioreactor.

Preferably the oscillatory flow has a flow rate between 0.01 ml/min and 50 ml/min, preferably from 0.03 ml/min to 3 ml/min.

The present invention also provides a cellularized or recellularized pericardial tissue obtainable from the *ex vivo* method as defined above.

The present invention also provides the use of the cellularized or recellularized pericardial tissue for the production of heart valve leaflet, preferably for the manufacture of conventional and mini-invasively implantable valve prostheses or for use in surgical reconstruction of an aortic valve and/or a mitral valve.

The present invention also provides an *ex vivo* method for producing a heart valve leaflet or for producing a cellularized or recellularized blood vessel, said leaflet or said blood vessel comprising autologous or heterologous cells selected from the group consisting of: valve-derived interstitial cells, adipose-tissue stromal/mesenchymal cells, myocardial stromal/mesenchymal cells, Wharton Jelly stromal/mesenchymal cells and vascular-derived stromal/mesenchymal/progenitor cells or a mixture thereof, said method comprising the steps of:

a) introducing said autologous or heterologous cells into the decellularized pericardial material as defined above by means of an oscillatory flow thereby producing a cellularized pericardial tissue;
b) cutting and/or trimming the cellularized pericardial tissue, preferably by means of a valve leaflet sizer, preferably to form a blood vessel.

The present invention also provides a heart valve leaflet or a cellularized or recellularized blood vessel obtainable by the method as defined above.

The present invention also provides the decellularized pericardial material as defined above, the cellular pericardial tissue as defined above or the heart valve leaflet or the cellularized blood vessel as defined above for use in surgery or for medical use, preferably for use in the treatment of diseased and/or damaged heart tissue, preferably said diseased and/or damaged heart tissue is a calcific aortic valve disease, aortic valve insufficiency, mitral prolapse, mitral stenosis, preferably said surgery is an implantation of a heart valve, a replacement of a heart valve, a repair of a heart valve or a heart valve limb reconstruction, preferably said heart valve leaflets reconstruction is an Ozaki procedure, a Ross Procedure, preferably the decellularized pericardial material as defined above, the cellular pericardial tissue as defined above or the heart valve leaflet or the cellularized blood vessel as defined above is for use in the treatment of vascular disease, preferably the vascular disease is a peripheral and/or coronary artery disease, preferably the decellularized pericardial material as defined above, the cellular pericardial tissue as defined above or the heart valve leaflet or the cellularized blood vessel as defined above is for use for replacement of a peripheral and/or coronary artery in peripheral and/or coronary bypass grafting.

In the present invention, the term "decellularize" refers to removal of any cell content and xenoantigens from pericardium by means of a sequential incubation into decellularization solutions, as tested by means of immunofluorescence, DNA quantification methods, proteomics and mass spectrometry-based approaches.

In the present invention, the term "recellularize" or "cellularized" refers to introduction of cells into the decellularized pericardium by means of bioreactor-based or passive culture, as tested by means of immunofluorescence, DNA quantification methods, proteomics and mass spectrometry-based approaches.

The expression "oscillatory flow" as used herein refers to the modality of cell introduction inside the decellularized tissue with an alternate motion of medium, fluid or buffer in both directions across the tissue.

The expression "living tissue incorporating cells" refers to a tissue into which cells are introduced by a passive or active cellularization method promoting, helping or forcing cell penetration.

The invention will be now illustrated by means of non-limiting examples referring to the following figures.

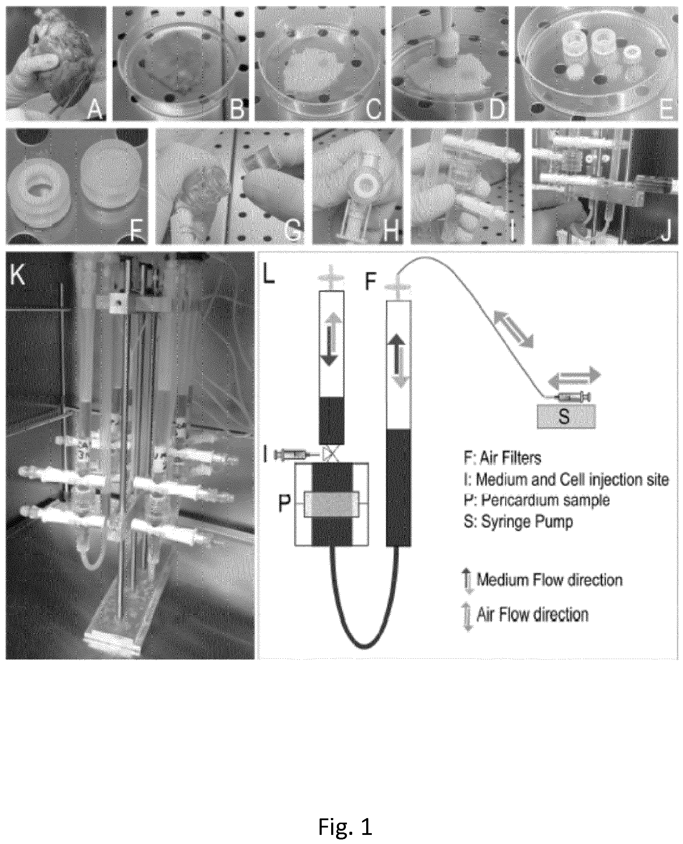

**Figure 1. Description of the pericardium processing.** After the dissection from the pig hearts left ventricles (A), the parietal pericardium was further dissected and decellularized as described in the materials and methods (B-C). It is evident the change in the color of the tissue due to removal of blood and cell content. Patches with a 1cm diameter of were cut from the tissue with a biopsy puncher (D) and then mounted into the bioreactor holders (EH). After joining the holders to the bioreactors circuits (I), the assembled bioreactors were placed onto a rack for transfer into the culture incubator, and connection to the peristaltic pump (J-K). Note in panel J the injection of the cells into the bioreactor that was operated by a syringe. (L) Schematic representation of the bioreactor working principle. The pericardium sample is clamped in the chamber, allowing medium to flow only through the patch. Culture medium and cell suspension are injected through the injection site, and the alternate reverse flow is controlled by a syringe pump.

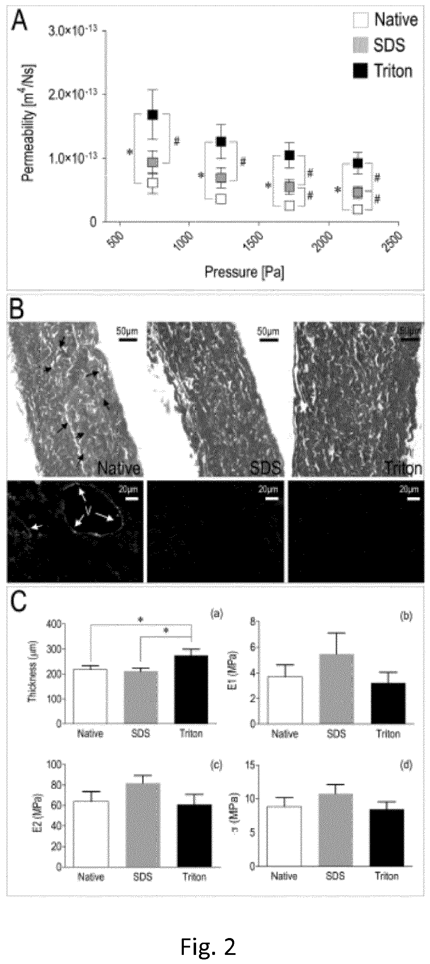

**Figure 2. Permeability, structural, antigenic and mechanical characterization of the pericardium.** (A) Permeability of the tissue was plotted as a function of the applied pressure. The SDS-based decellularization determined an increase in permeability of the tissue compared to the untreated pericardium only at the highest pressure levels. By contrast, the Triton-treated pericardium exhibited higher values at all the considered pressures compared to Native or SDS-decellularized samples. Statistical significance of grouped data comparison was determined by two ways ANOVA with Bonferroni post-hoc analysis (P < 0.05 indicated by *, n=8) and by pairwise Student's t-test for comparisons at each pressure level (P < 0.05 indicated by #, n=8) (n=8). Data are plotted as means with standard errors. (B) Histological appearance and antigenic characterization of Native, SDS-treated and Triton-treated pericardium. Upper panels show the results of Verhoeff/Van Gieson staining of the tissues (arrows indicate cell nuclei), while the lower panels the immunofluorescence staining with the nuclear dye DAPI and an antibody recognizing the pig $\alpha$GAL (Santoro, et al., 2016). Note the complete removal of the nuclei in the decellularized samples in all panels, and that of the major pig xenoantigen (green fluorescence) in the lower. (C) The morphometric evaluation of the samples thickness (graph a) revealed a significant increase in the Triton-treated samples compared to the other two conditions, possibly due to increase in tissue porosity and permeability. Data (n=10 donors) are plotted as means with standard errors. Mechanical characterization by UTL tests (graphs b-d) allowed to determine the resistance to traction of the native and the decellularized samples. None of the parameters in these tests differed significantly between treatments. This, in keeping with our previous results (Santoro, et al., 2016, Vinci, et al., 2013) indicates that the two decellularization methods did not affect pericardium mechanical characteristics. * indicates by P < 0.05 by one way ANOVA (repeated measures) with a Newman Keuls post-hoc comparison (n=10). Stress was calculated dividing the machine measured force by the resistant area (thickness times width). Strain was calculated by normalizing the machine actuator displacement by the specimen initial length. From the resulting stress-strain curves ($\sigma$-$\epsilon$), the biomechanical properties of the pericardial tissue were described by means of elastic modulus (the $\sigma$-$\epsilon$, curve slope) at low (E1) and high (E2) strain, as well as the maximum tensile stress ($\sigma$max).

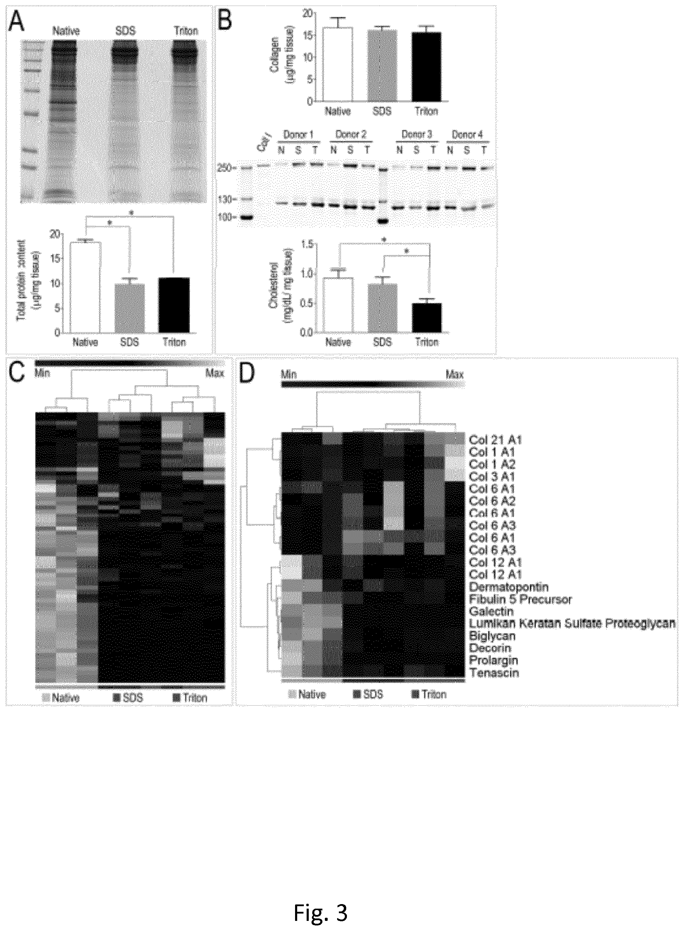

**Figure 3. Protein and lipid analysis of decellularized pericardium.** (A) SDS-PAGE of total proteins extracted from native, SDS- and Triton-decellularized pericardium. As it is evident by the electrophoretic run and the protein quantification by Colloidal Blue, a similar extent proteins removal (especially in the low molecular weight range) was observed by both decellularization methods. * indicates P < 0.05 by one way ANOVA (repeated measures) with a Newman Keuls post-hoc comparison (n=3). (B) Collagen content evaluation performed by Sircol (upper graph) and Western Analysis (middle picture) showed that the two decellularization methods did not impact on the overall collagen content. By contrast, the cholesterol content was sharply reduced by the Triton-, but not the SDS-, based decellularization method. * indicates by P < 0.05 by one way ANOVA (repeated measures) with a Newman Keuls post-hoc comparison (n=4 and 6 for Sircol red collagen determination and cholesterol content, respectively). (C) The heatmap shows the results of the unsupervised clusterization analysis of all the detectable proteins by quantitative mass-spectrometry analysis. This showed a clearly different protein representation in SDS and Triton-treated vs. the untreated samples. The analysis indicated the existence of two clusters in which proteins were overrepresented in decellularized vs. native samples and vice versa. (D) Graphic representation of the clusterized data of the protein relative abundance representing extracellular matrix components (details in Table 1), as detected by mass-spectrometry in pericardial samples. The protein identity is indicated according to ENSEMBL and UNIPROT databases annotation. The heatmap shows that decellularization treatment caused a variation in the abundance of matrix-specific proteins, with a substantial elevation of the collagen species (except for Collagen12), and an abatement of the proteoglycan content.

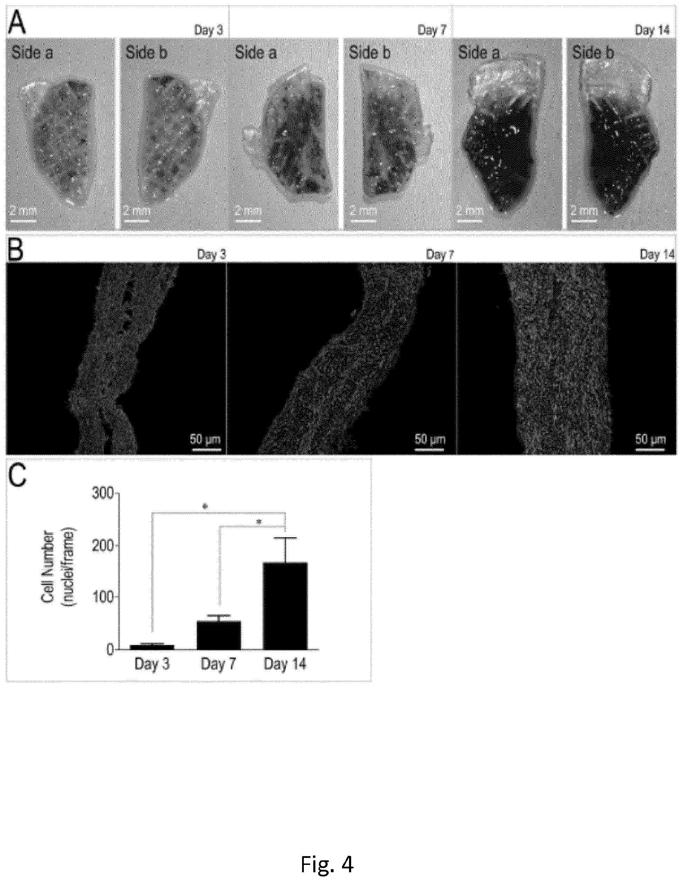

**Figure 4. Adoption of a bioreactor-based perfusion cell seeding enhances cellularization efficiency and growth of VICs in Triton-treated pericardium.** (A) MTT staining of semilunar Triton-treated pericardial patches cellularized and matured in perfusion bioreactor. The increment in MTT color staining on both sides of the patches denotes a substantial growth of cells over the time of tissue culture. (B-C) DAPI staining in transversal sections of recellularized patches was employed to assess VICs growth inside the recellularized tissue. Cells quantification denoted a robust and constant increment of the cell number in the patches. * indicates by P < 0.05 by one-way ANOVA (repeated measures) with a Newman Keuls post-hoc comparison (n=4).

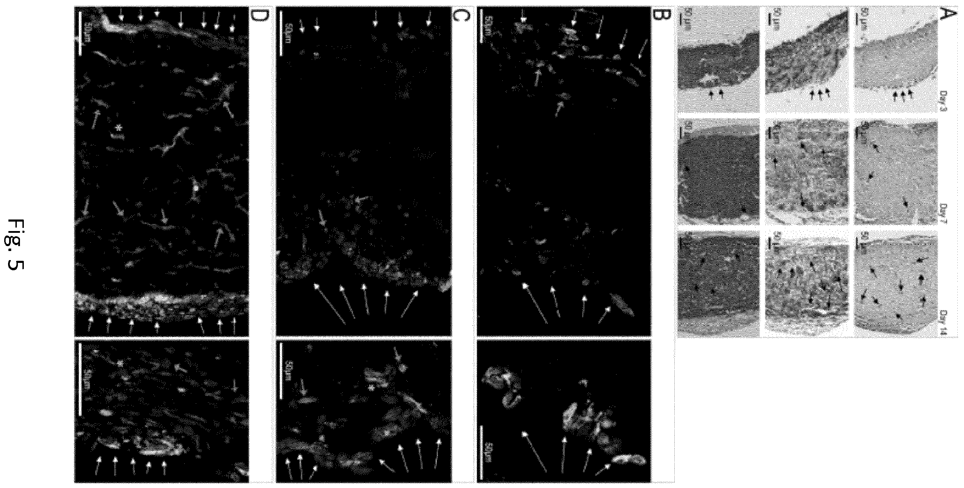

**Figure 5. Histological and immunofluorescence-based characterization of the recellularized pericardium patches.** (A) Histological appearance of the Triton-treated recellularized pericardium during the course of maturation in the perfusion bioreactor as detected by Hematoxylin/Eosin, Masson's Trichrome and Verhoeff/Van Gieson staining (upper, middle and lower panels, respectively). The pictures show that cells progressively invade the tissue from the two surfaces. Note that none of the staining methods revealed a change in the structure of the collagen fibers at any time during the course of tissue maturation inside the culture system. (B-D) Low and high magnification of confocal-based imaging of porcine VICs colonizing the pericardium at different culture time points (panel B: 3 days; panel C: 7 days; panel D: 14 days). Sections were stained with antibodies recognizing Vimentin (red fluorescence) and $\alpha$SMA (green fluorescence). As shown in the panels, the culture into the oscillating perfusion system determined a progressive increase in the cell content inside the tissue. Interestingly, cells differed for the expression of $\alpha$SMA depending on their position in the constructs, with high levels in cells adhering to construct surface (white arrows) and low or absent level in cells found inside the tissue (blue arrows). This latter finding suggests that VICs undergo a transition from an activated to a quiescent phenotype when surrounded by pericardium matrix.

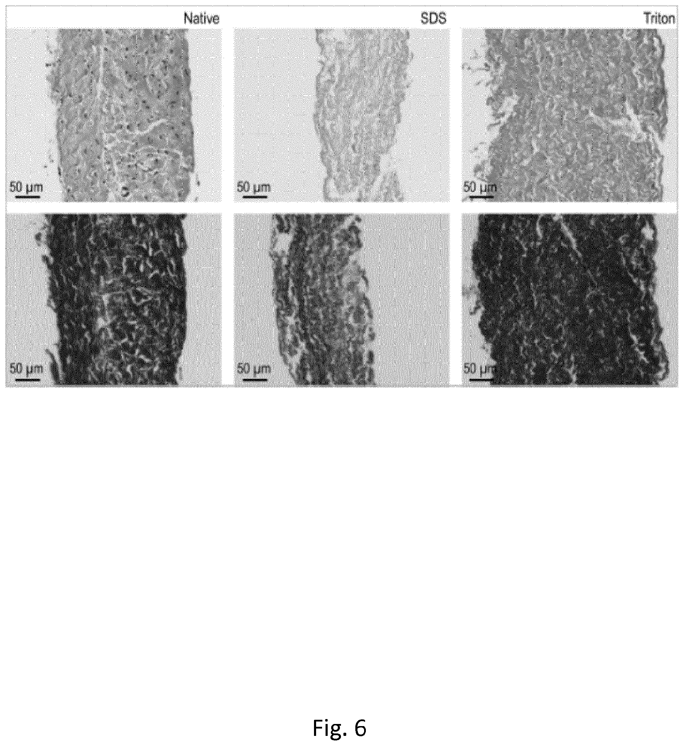

**Figure 6. Histological appearance of native** *vs.* **SDS- and Triton-treated pericardium.** Panels show the Hematoxyilin/eosin and Masson's trichrome staining of similar sections to those stained with Verhoeff/Van Gieson included in Figure 2B. Note the complete absence of cells in the decellularized samples, especially in Triton-treated samples and the absence of morphological alteration in the matrix structure of the tissue.

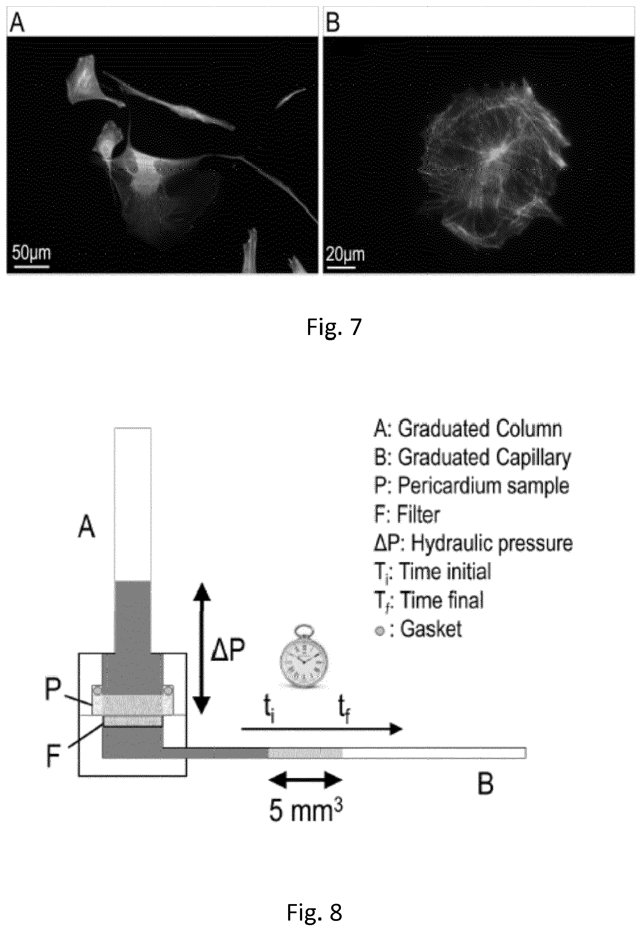

**Figure 7. Expression of SMA and Vimentin into porcine Ao-VICs cultured onto plastic TC dishes. Panel A** represents cells stained with Phalloidin-TRITC (red fluorescence) to visualize the F-Actin cytoskeleton and anti-SMA antibody (green fluorescence). **Panel B** shows a high magnification of one porcine Ao-VIC stained with Phalloidin (red) and anti-Vimentin antibody (green fluorescence).

**Figure 8. Scheme representing the functioning of the apparatus employed to monitor pericardium permeability.**

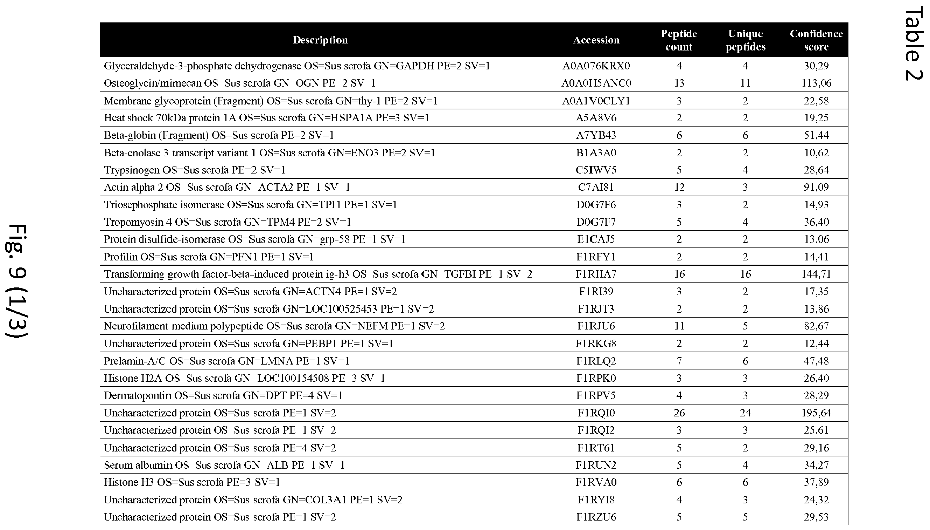

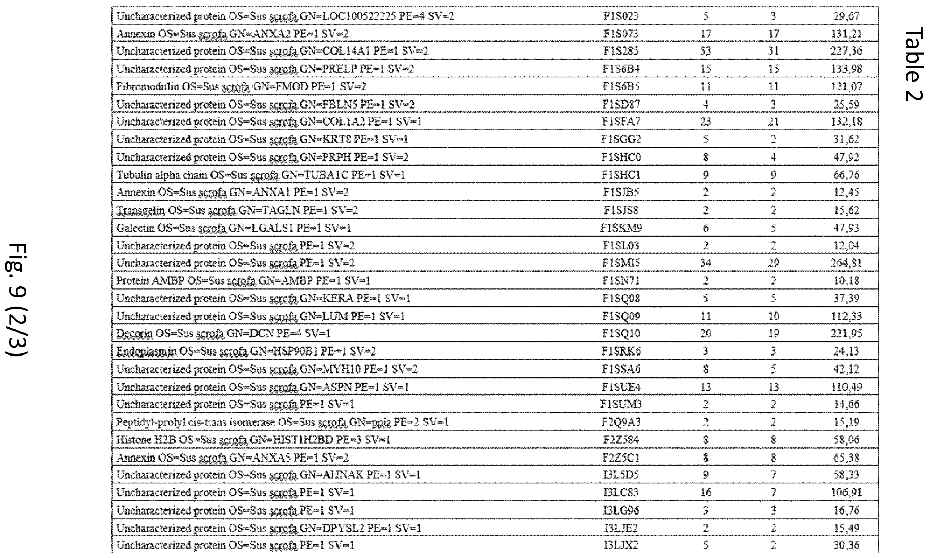

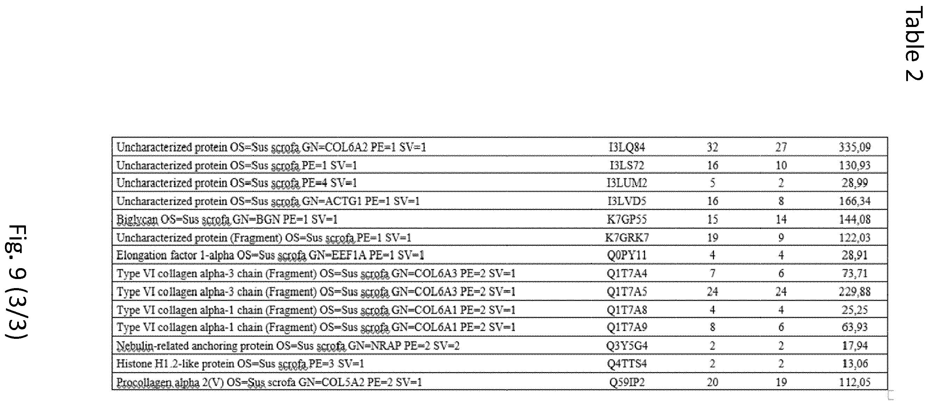

**Figure 9. Table 2:** Complete List of the proteins identifiable by mass spectrometry.

## DETAILED DESCRIPTION OF THE INVENTION

### Materials and Methods

*Porcine pericardium decellularization procedures*

Parietal pericardial tissue was dissected from the left ventricle of pig hearts ($10 \pm 2$ months of age) donated by a local slaughterhouse. Two different decellularization procedures were used and compared. The first, named "SDS", was previously described by us (Santoro, et al., 2016), the second, named "Triton", is a refinement of this protocol. Briefly, both procedures start with a first mechanical removal of fat tissue, followed by three 30 min washings in Ca2+ and Mg2+ free phosphate-buffered saline (PBS) containing protease inhibitors (Aprotinin, 10 KIU mL-1, Trasylol, Bayer, Germany) under continuous agitation. Cell lysis was achieved by osmotic shock treating the tissue with hypotonic buffer (10 mM Tris-HCl; pH 8.0), supplemented with protease inhibitors (Aprotinin, see above), for 16 h, under continuous agitation at 4°C. Samples following the "Triton" protocol were additionally incubated for 24 h in 1% (v/v) Triton X-100 in bi-distilled water at room temperature under continuous agitation, aiming to break lipid-lipid and lipid-protein interactions. To ensure cell debris removal, both "SDS" and "TRITON" samples were repeatedly washed (3 washes, 30 minutes each, in agitation) with bi-distilled and incubated for 24 h in 0.1% (w/v) sodium dodecylsulphate (SDS) in bi-distilled at room temperature under continuous agitation. 90 minutes washings in bi-distilled, were followed by a 30 min washing in PBS with Ca2+ and Mg2+ before incubation for 3h at 37°C in a reaction buffer containing 50 U mL-1 deoxyribonuclease I from bovine pancreas (DNase, Sigma-Aldrich, Germany) under agitation, serving to nucleic acid material removal. After washing in bi-distilled for either 24 (Triton) or 48 (SDS) hours under agitation, samples were, finally, incubated for 72 hours at 4 °C in BASE 128 (AL.CHI.MIA S.r.1.), an EC certified decontamination medium containing an antibiotic/antifungal mixture (Gentamicin, Vancomycin, Cefotaxime and Amphotericin B) approved for employment in Tissue Banking (Gatto, et al., 2013). Mechanical response and biochemical composition of SDS- and Triton-treated decellularized samples were then compared vs. the native tissue. Finally, 10 mm diameter circular patches of decellularized pericardium were cut using a biopsy puncher and used for static or dynamic VICs seeding and culture.

*Mechanical characterization*

Uniaxial tensile loading (UTL) tests were performed on Native, SDS- and Triton-decellularized pericardium from 10 donors (at least 2 samples per donor). To assess mechanical characteristics rectangular shaped specimens of 3 mm width were dissected with no preferential orientations, assuming pericardium isotropy. The ends of the specimens were glued with cyanoacrylate adhesive in between of two sheets of sandpaper, to facilitate uniform gripping and to avoid sample slippage. After mounting onto the clamps of the testing machine (MTS System Corporation), samples were preloaded up to 0.01 N and preconditioned through loading-unloading cycles (6 cycles) at 15% maximum strain with an elongation rate of 10 mm/min, until the loading-unloading curves were almost superimposed. After tissue preconditioning, the specimens were preloaded up to 0.01 N. The specimen initial length was measured and UTL test was conducted, at a constant velocity of 10 mm/min, until specimen failure. UTL tests were performed at room temperature and the specimens were maintained hydrated with PBS for the whole test duration. The thickness of each specimen was measured by histological analysis from samples collected next to those used for mechanical tests. Stress was calculated dividing the machine measured force by the resistant area (thickness times width). Strain was calculated by normalizing the machine actuator displacement by the specimen initial length. From the resulting stress-strain curves ($\sigma$-$\varepsilon$), the biomechanical properties of the pericardial tissue were described by means of elastic modulus (the $\sigma$-$\varepsilon$ curve slope) at low (E1) and high (E2) strain, as well as the maximum tensile stress ($\sigma$max).

Perfusion tests were employed to evaluate and compare permeability properties of the tissues from 8 donors.

The apparatus used to perform the test is custome made. It is constituted of 2 coaxial stainless steel cylindrical parts, and a capillary flow-meter with a resolution of 1 µl. Round-shaped tissue specimens, maintained in PBS at 4°C until the test, were housed into the lower cylinder, over a polyethylene porous filter, and secured between two gaskets. Fluid pressures ranging from 0.74 to 2.21 MPa were obtained using a vertical column filled with different volumes of PBS. After tissue equilibration, time required to filter 5.00 mm3 for each pressure was measured and the permeability factor K was calculated as defined by the Darcy's Law.

$$K = \frac{Q * L}{A * \Delta P}$$

*Cell and static tissue Culture*

Primary aortic valve interstitial cells (VICs) were obtained by enzymatic isolation from explanted porcine aortic valves (10±2 months of age) as previously described (Santoro, *et al.,* 2016a). Cells were cultured up to four passages in standard culture dishes, in DMEM (Lonza) containing 150 U mL$^{-1}$ penicillin/streptomycin (Sigma-Aldrich), 2mM L-glutamine (Sigma-Aldrich) and 10% fetal bovine serum (HyClone, Thermo Scientific). For static culture, the 10 mm diameter decellularized pericardium samples were fixed to silicone holders and housed into 6 wells plates. 1.50 x 10$^5$ cells were seeded onto each sample, by gently pipetting 50 µl of cell suspension. 2 hours after seeding, 2 ml of complete medium were added to the well, and culture was performed up to 7 days, before dividing each sample in 2 halves, respectively, to perform MTT staining or immunofluorescence staining of cross-sections, as described below.

*Bioreactor culture*

After the decellularization procedure, the pericardium was washed abundantly with sterile PBS to ensure BASE.128 removal. 10 mm diameter patches were obtained using sterile biopsy puncher and were housed into bioreactor chambers, by means of a silicone holder (Figure 1), leaving 8 mm diameter of the sample free to allow fluid flow and preventing lateral leakage. After assembly, 10 ml of cell suspension, containing 6.5 E+05 VICs, were injected into each bioreactor using the "injection site" as shown in Figure 1L. Oscillatory flow was then started. Flow rate during this phase was optimized in preliminary experiments performed on SDS pericardium, by applying a seeding flow rate of 0.03 mL min-1, 3 mL min-1 and 6 mL min-1 for 24 hours. On the basis of these results, recellularization procedure was established. This consisted in an initial seeding phase (72 hours) at a high (3 mL min-1) flow rate, followed by a culturing phase, lasting up to 14 days, at a low flow rate (0.03 mL min-1). During cell seeding and the following culturing phases, a reversing flow protocol was applied. This consists in producing an alternate motion of 3 ml medium in both directions across the tissue (Figure 1L), that ensures to maintain the patches always in contact with medium and to maximize gas/nutrient supply. Culture medium was partially (6 mL) substituted every 72 hours. At the end of the period, dynamically recellularized samples were harvested, and either destined to MTT assay, or fixed to perform histological sectioning. MTT staining was performed by incubating samples in 400 µL of 1.2 mM MTT [3(4,5- dimethylthiazol-2-yl)-2,5-diphenyltetrazolium bromide] solution at 37°C for 3 h. After incubation, images of both sides of the in vitro cellularized pericardium were acquired with Stemi 2000-C Stereo Microscope (Carl Zeiss, Germany).

*MTT staining assay*

MTT staining was performed by incubating samples in 400 µL of 1.2 mM MTT [3(4,5-dimethylthiazol-2-yl)-2,5-diphenyltetrazolium bromide] solution at 37°C for 3 h. After incubation, images of both sides of the *in vitro* cellularized pericardium were acquired with Stemi 2000-C Stereo Microscope (Carl Zeiss, Germany).

*Histological sectioning, staining, and immunofluorescence*

Pericardium samples, before and after decellularization/recellularization, were fixed in 4% PFA and embedded in paraffin. Histological sections (4 µm thick) were cut and subsequently dewaxed and stained with either Verhoeff-Van Gieson, Hematoxylin and Eosin (H&E) or Masson's trichrome staining and acquired using an Axioscop optical microscope (Carl Zeiss, Germany). Cell distribution in the pericardium was assessed by DAPI (Sigma-Aldrich) nuclei staining. Cell counting was performed by a semi-automated method using ImageJ Software (National Institute of Health, USA). Immunofluorescence analysis were performed on recellularized samples, following unmasking procedure with Sodium Citrate Buffer (10mM, 0.05% Tween-20, pH6.0) at 100°C, permeabilizeation (1% v/v Triton X-100 in PBS-BSA 3% w/v) and incubated with anti-Vimentin (Rabbit, Cell Signalling) and anti-aSMA (Mouse, DAKO) antibody for 16 hours at 4°C. After incubation with secondary antibodies (AlexaFluor 633 and AlexaFluor 488, Life Technologies) for 1 hour at room temperature and DAPI (DAKO) counterstaining, images were acquired with an Apotome fluorescence microscopes (Carl Zeiss, Germany) and Laser Scanning Confocal Microscope (Carl Zeiss, Germany).

*Biochemical characterization and mass spectrometry analysis*

Lipids were extracted from homogenized native and decellularized pericardium in TNES buffer using the Folch method (Folch, et al., 1957). Cholesterol concentration was measured using an enzymatic assay (Allain, et al., 1974) and normalized for dry tissue weight. For total protein quantification, an extraction buffer containing Tris 0.1M Ph 7.6 and SDS 4% was used. Homogenization was performed with Ultraturrax at 24000 rpm for 30 seconds on ice followed by sonication (10 seconds twice power 6 on ice) and heating at 95°C for 3 min. Equal amounts of proteins from Native, SDS and Triton samples (15ug) were separated by 12% polyacrylamide gel electrophoresis after dilution in Laemmli buffer (Laemmli, 1970) and stained with the Colloidal Blue method (Banfi, et al., 2009). For label-free quantitative mass spectrometry (LC-MSE), the protein extracts were precipitated with the Protein precipitation kit (Calbiochem, MerckMillipore, Milan, Italy), according to the manufacturer's instructions. Protein pellets were then dissolved in 25 mmol NH4HCO3 containing 0.1% RapiGest (Waters Corporation, Milford, MA, USA) and digested as previously described (Brioschi, et al., 2013). LC-MSE analysis was performed as previously described and analyzed using Progenesis QIP including normalization of protein abundance considering all the identified proteins (Brioschi, et al., 2014, Roverso, et al., 2016).

Collagen quantification was performed by Sircol soluble collagen (Biocolor) colorimetric assay. Portions (around 45 mg) of frozen tissue (4 donors) were reduced in small pieces by Tissue Pulverizer (Bessman), then collected, weighted and digested over-night at 4 °C in 1 mL of pepsin 0.1 mg/ml and 0.5 M acetic acid solution. The colorimetric reaction was analyzed with absorbance reading at 555 nm on Tecan Infinite M200 PRO spectrophotometer. Absolute values were calculated from interpolation with a curve generated by an internal standard and normalized for dry tissue weight (mg). For Western Blot Analysis, tissue slices from 4 different donors were weighted, minced into small pieces (around 1 mm3) and homogenized with Ultraturrax in Tris 0.1M Ph 7.6 and SDS 4% buffer as described above. After the sonication, samples were centrifuged at 13000 rpm for 10 minutes and the supernatant was collected. Following protein concentration determination (Pierce™ BCA Protein Assay Kit, Thermo scientific), 2.5 $\mu$g of total protein solution into Laemmli buffer was heated at 95°C and run into a pre-casted 4-12% gradient polyacrylamide gel (BioRad). Collagen Type I solution (2.5 $\mu$g) from rat tails (Sigma) was loaded and run as a positive control. Proteins were then transferred to a nitrocellulose membrane (25 mM Tris, 190 mM Glycine, 20% Methanol, SDS 0.02% for 2h at 100 V). Non-specific binding was blocked by incubation with 5% non-fat dried milk in PBS. Overnight incubation at 4 °C with anti-Collagen primary antibodies (Rabbit, Abcam) was followed by incubation for 20 minutes at room temperature with secondary antibodies (LI-COR). Band densitometry was acquired by Odyssey (LI-COR).

*Statistical analysis and data clusterization*

All values in bar graphs are represented as mean $\pm$ standard error. Differences among experimental groups were assessed by Graph-Pad statistical software. The type of statistical tests and the number of the replicates included in the analyses are specified in the figure legends. Proteomics data clusterization was performed by MeV software with the normalized protein abundance data.

## EXAMPLES

*Example 1: Definition of an optimized pericardium decellularization process for valve tissue engineering applications*

Our previous investigations showed that treatment of pericardium with decellularizing agents abolishes the immunological rejection into an immunocompetent environment (Vinci, et al., 2013), and is permissive for cell culturing using valve-derived interstitial cells (Santoro, et al., 2016). Since static cell seeding does not, in general, allow a consistent and reproducible colonization, especially in the inner layers (Dainese, et al., 2012, Namiri, et al., 2017), the inventors speculated that the adoption of a perfusion bioreactor (Wendt, et al., 2003a) might improve efficiency and reproducibility of the process. In the initial part of the work, the inventors therefore designed the strategy to drive cells in the whole pericardium according to conditions already established for synthetic fibrous scaffolds (Santoro, et al., 2010). A direct perfusion commercial bioreactor (U-CUP) was chosen to this aim (Wendt, et al., 2003a). Indeed, the working principle and the performance of this system have been extensively described to enable cell seeding into three-dimensional scaffolds by continuous perfusion of a cell suspension through the scaffold pores. The oscillating direction of the culture medium is automatically controlled by the movement of a syringe pump. Based on the same principle, homogeneous culture conditions and mass transfer (gas and nutrients supply and waste removal) are guaranteed in the whole scaffold volume during long term culture. Figure 1 shows the phases of tissue preparation from the dissection of the LV parietal pericardium (A-B) to the cutting of the 1 cm diameter patches (D-E) to be mounting into the holders (F), and final bioreactor assembly (G-K). A schematic representation of the bioreactor working principle is provided into panel L.

For setting the VICs cellularization method, the inventors used pericardium decellularized with the SDS-based protocol previously published by us (Santoro, et al., 2016). The MTT staining of the recellularized patches exhibited a uniform cell distribution on both sides of the patches only in samples that were subject to a 3 ml/min flow rate. On the other hand, the lack of a sufficient cell number in the inner layers prolonging culture time up to 7 days (not shown) suggested a sub-optimal seeding efficiency, likely due to an insufficient permeability of the tissue. In line with this hypothesis, permeability tests showed an insufficient permeability of the SDS-based decellularized tissue that resulted more permeable compared to native tissue only at the highest tested pressures (Figure 2A). In order to increase the permeability of the tissue, thus making it more suitable to be perfused, the inventors set a new decellularization protocol, which included incubation with TritonX-100, a non-ionic detergent that specifically breaks the lipid-lipid and lipid-protein interactions without denaturing the extracellular matrix (Seddon, et al., 2004), after the SDS phase. Figure 2A shows that the addition of TritonX-100 to the decellularization procedure determined an increase in the permeability of the tissue vs. Native controls at all the tested pressures (fold change $5.08\pm2.33$; $6.05\pm2.93$; $6.66\pm3.17$; $7.56\pm3.54$, mean$\pm$SE, n=8, from lowest to highest pressure values as indicated in the figure, respectively). The enhancement in tissue permeability induced by TritonX-100 incubation caused, as estimated by the Darcy low, a one-fold decrease in the hydraulic velocity in the pericardium, both during seeding and culture phases. To monitor the structure of the Triton-treated pericardium, a histological evaluation of the tissues was performed by conventional staining methods. Results of Verhoeff-Van Gieson (Figure 2B), hematoxylin/eosin and Masson trichrome staining (Figure 6) showed a substantial structural integrity of the matrix fibers in Triton-decellularized vs. the two other conditions, although the tissue appeared more swollen, consistently with a higher porosity. In keeping with these results, thickness of the samples treated with the Triton-containing procedure was higher than that of the two controls (fold change $0.99\pm0.07$ and $1.31\pm0.08$; n=10 for SDS and Triton treatments vs. Controls, respectively; Figure 2C). Finally, to assess whether the addition of Triton to decellularization procedure compromised the tissue mechanical performance, the inventors conducted Uniaxial Tensile Loading (UTL) tests. The three parameters (E1, E2 and $\sigma$max) that were derived from traction tests showed a similar behavior in all tested conditions (Figure 2C).

*Example 2: Biochemical characterization of the decellularized characterization reveals complete removal of cellular proteins without affecting extracellular matrix components*

The previous results showed that addition of an incubation step with Triton to the SDS-based decellularization method increased permeability without jeopardizing the structure and the mechanical resistance of the pericardial matrix. On the other hand, since the composition of the extracellular matrix is crucial to ensure an optimal growth of valve cells, the inventors investigated the effects of the decellularization method on the total proteins and collagen content. The inventors completed this analysis with the assessment of the lipids components and a quantitative mass spectrometry-based investigation. The results of total protein quantification indicated a significant decrease into either SDS- and Triton-based decellularization methods content, especially in the low molecular weight component (Figure 3A). This decrease was similar in Triton- and SDS-based treatments, suggestive of a similar efficiency of the two methods at removing cellular proteins.

The analysis of collagen content by Sircol Red and Western blotting showed that the total amount of collagen I, the main pericardium matrix component, was not reduced by none of the treatments (Figure 3B). Furthermore, the mass spectrometry approach followed by unsupervised clusterization analysis (Figures 3C and D) indicated a change in the relative protein composition between the untreated and the treated samples. This determined an increase in the relative amount of collagenous structural matrix component (with exception of Col14A1), and a parallel reduction of cellular proteins and proteoglycans matrix component (see Tables 1 and 2 (Fig. 9) for data on relative abundance of the matrix related protein components and description of the identifiable proteins, respectively).

EP 3 533 475 A1

**Table 1**. Normalized abundance (NA: mean and standard deviation) of the extracellular matrix-related proteins identified by mass spectrometry in Native, SDS- and Triton-treated pericardial samples (data also clusterized in **Figure 3D**). Protein identity is indicated according to ENSEMBL/UNIPROT databases. Data are representative of 3 independent donors. Pairwise comparisons significance was calculated by ANOVA using the Progenesis Software.

| Accession | Protein ID | Native (mean NA) | Native (S.D) | SDS (mean NA) | SDS (S.D.) | Triton (mean NA) | Triton (S.D.) | Native vs. SDS | Native vs. Triton | SDS vs. Triton |
|---|---|---|---|---|---|---|---|---|---|---|
| K7GP55 | Biglycan | 16863.1 | 2501.2 | 8261.6 | 1429.6 | 4580.7 | 732.8 | <0.0001 | <0.0001 | <0.0001 |
| I3LJX2 | Col 1 A1 | 1676.1 | 324.8 | 1954.2 | 168.2 | 2383.4 | 494.6 | <0.05 | <0.001 | n/s |
| F1SFA7 | Col 1 A2 | 12986.9 | 2061.9 | 14074.0 | 1346.0 | 17413.4 | 4199.9 | n/s | <0.001 | n/s |
| F1RQI0 | Col 12 A1 | 15959.8 | 6935.0 | 12952.7 | 1646.1 | 10061.5 | 1651.6 | n/s | <0.05 | <0.001 |
| F1RQI2 | Col 12 A1 | 1665.2 | 1061.8 | 981.2 | 378.2 | 742.6 | 150.2 | n/s | n/s | n/s |
| F1RZU6 | Col 21 A1 | 1189.8 | 209.1 | 1043.0 | 211.9 | 1288.6 | 422.0 | n/s | n/s | n/s |
| F1RYI8 | Col 3 A1 | 4462.9 | 911.0 | 4552.4 | 621.3 | 6146.3 | 1711.3 | n/s | n/s | <0.01 |
| I3LS72 | Col 6 A1 | 8741.7 | 1350.8 | 9572.7 | 1265.6 | 8814.4 | 1224.5 | n/s | n/s | n/s |
| Q1T7A8 | Col 6 A1 | 1903.1 | 461.2 | 3325.3 | 246.1 | 2623.1 | 602.5 | <0.0001 | <0.01 | <0.01 |
| Q1T7A9 | Col 6 A1 | 4092.8 | 740.6 | 6606.2 | 1046.6 | 5773.3 | 860.3 | n/s | n/s | n/s |
| I3LQ84 | Col 6 A2 | 26275.6 | 4568.9 | 33652.1 | 4812.7 | 29312.9 | 4881.8 | n/s | n/s | n/s |
| Q1T7A4 | Col 6 A3 | 6906.2 | 1356.2 | 10895.4 | 2267.4 | 9463.6 | 1165.9 | n/s | n/s | n/s |
| Q1T7A5 | Col 6 A3 | 15785.6 | 3517.3 | 24473.7 | 932.4 | 20734.4 | 3254.1 | n/s | n/s | n/s |
| F1SQ10 | Decorin | 36435.2 | 7098.8 | 20054.4 | 2431.5 | 11143.4 | 690.2 | <0.0001 | <0.0001 | <0.0001 |
| F1RPV5 | Dermatopo ntin | 1583.7 | 288.1 | 1180.3 | 147.1 | 779.7 | 198.2 | <0.001 | <0.0001 | <0.001 |
| F1SD87 | Fibulin 5 Precursor | 1002.1 | 184.7 | 610.7 | 117.5 | 431.3 | 184.2 | <0.0001 | <0.0001 | <0.01 |
| F1SKM9 | Galectin | 1980.5 | 143.8 | 31.0 | 5.0 | 3.8 | 0.9 | <0.0001 | <0.0001 | <0.0001 |
| F1SQ09 | Lumikan Keratan Sulfate Proteoglyca n | 20751.7 | 2108.5 | 1809.7 | 149.5 | 1297.1 | 383.8 | <0.0001 | <0.0001 | <0.01 |
| F1S6B4 | Prolargin | 17213.9 | 6219.9 | 1345.0 | 163.4 | 927.7 | 104.0 | <0.0001 | <0.0001 | <0.0001 |
| F1SMI5 | Tenascin | 13245.4 | 2673.1 | 5557.9 | 2265.8 | 9464.1 | 967.8 | <0.0001 | <0.0001 | <0.0001 |

Finally, the assay of cholesterol content revealed a significantly lipid decrease only in Triton-treated samples (Figure 3B). Taken together, these results suggest that Triton-based decellularization does not affect the major structural and mechanical component of the tissue but determines an increased tissue permeability by efficient lipids removal compared to the SDS treatment.

*Example 3: Perfusion-driven seeding ensures physiologic growth of valve interstitial cells inside the decellularized pericardium*

The lower local velocity in the inner layers of the pericardium was supposed to favor a more homogeneous and efficient cell seeding, by better supporting cell adhesion and survival, and reducing the shear stress on the cells, without jeopardizing mass transfer inside the tissue. A final recellularization procedure was therefore established including an initial VICs oscillatory seeding phase for three days at 3 ml/min flow rate (v=1 mm/s), followed by a tissue maturation phase (still under oscillatory flow) at 0.03 ml/min (v=10 μm/s) (Santoro, et al., 2012). Under these conditions, according to MTT staining, a constant VICs increase occurred inside the entire pericardium volume (Figure 4A). The analysis of transversal tissue sections of the recellularized Triton-treated pericardium, followed by nuclei counting, confirmed a substantial and constant VICs growth (Figure 4 B-C). Remarkably, the presence of cells inside the tissue indicated the robustness of the recellularization protocol. To confirm the effectiveness of the recellularization process, an analysis of the VICs phenotype inside the recellularized tissue was performed by immunofluorescence. In this analysis, staining with antibodies recognizing Vimentin and αSMA, two markers of VICs and 'activated' VICs under standard culture conditions (Porras, et al., 2017) (Figure 7) were performed to discriminate between cells that may have a self-renewal vs. a pro-pathologic phenotype. Figure 5A shows representative images of the cells inside the pericardium at the three-considered time-points. In addition to reveal a robust growing of the cells that tended to progressively invade the tissue from the surface, an evident downregulation of the αSMA was observed, especially in cells colonizing the inner portion (Figure 5B). These results are consistent with a growth of the cells inside the decellularized matrix with a quiescent phenotype (Porras, et al., 2017).

**Discussion**

The results of the present invention show an unprecedented efficiency of cell seeding inside decellularized pericardium to generate fully cellularized valve-like tissue endowed with a self-renewal potential, thus setting a new standard in valve tissue engineering compared to existing literature. In fact, previous investigations showed insufficient cells growth throughout the tissue depth due to the limited cell penetration (Dainese, et al., 2012, Ghodsizad, et al., 2014, Liu, et al., 2016). In inventors' view, the key elements that contributed to these results were: the i) adoption of an optimized decellularization procedure based on sequential incubation of the pericardium into SDS followed by TritonX-100 solutions and ii) the employment of a standardized cellularization procedure based on oscillatory forced perfusion, which enabled a full penetration of cells inside the tissue, followed by a phase of tissue maturation.

In two previous reports published by inventors' group (Santoro, et al., 2016, Vinci, et al., 2013), a SDS-based decellularization process was adopted to remove cellular content and improve the immunocompatibility of human- and animal-derived pericardium for valve tissue manufacturing. While this method proved effective for removing xenoantigens and reduce the foreign body reaction after tissue transplantation, it was not yet thoroughly tested to produce an acellular scaffold suitable for valve engineering applications. Compared to the studies originally reporting the pericardium decellularization methods (Mirsadraee, et al., 2006, Mirsadraee, et al., 2007), the inventors focused their attention to demonstrate the acquisition of permeability by the tissue following decellularization. In fact, the inventors reasoned that only by enabling a substantial mass transport, able to vehicle cells and nutrients/oxygen inside the cell free tissue over the culture time, the inventors might achieve an effective colonization and maintenance of the cells in the inner layers. In one of their reports, in particular, the inventors already in part substantiated the observation that the SDS-based decellularization increases the permeability of the pericardium, even though the inventors did not test appropriately this issue (Santoro, et al., 2016). The results presented here (Figure 2A) extend those observations by showing that treatment with only SDS did not increase significantly the permeability of the pericardium if not at the highest applied pressures, thus making the tissue unsuitable to be efficiently recellularized at low pressure values. In keeping with this result, the bioreactor-based recellularization of the tissue decellularized with the SDS method was not efficient to ensure a robust penetration of the cells inside pericardium in a wide range of applied flow rates (not shown), thus calling for a further refinement of the decellularization method. The addition of an incubation step with a solution containing TritonX-100 helped the inventors to improve the decellularization efficiency by removing part of the lipids present in the tissue (Figure 3B). By destroying the interactions with proteins (Seddon, et al., 2004), the removal of lipids from the tissue caused a remarkable increase in permeability compared to native and SDS samples (Figure 2A). On the other hand, the evidence that the Triton method did not reduce the collagen content and the resistance to traction (Figures 2-3 and Table 1) shows that the method is potentially suitable to ensure a maximal resistance to mechanical stress typical of the opening/closing cycles of the natural valve. Thus, in addition to completely removing the α-GAL xenoantigen ((Santoro, et al., 2016) and Figure 2B), and reducing the propensity to induce calcification due to extensive lipids removal (Roosens, et al., 2016, Rossi, et al., 1990), the present method preserves the innate resistance of the pericardium tissue to mechanical stress and enables efficient cellularization with recipient-derived cells, which would not be rejected as non-self by recipient hosts.

As discussed in the previous sections, the increase in tissue permeability was only one of the two critical features to succeed in ensuring a substantial growth of valve derived-cells inside the pericardium. In fact, the seeding performed using simple static culture did not allow consistent and controllable VICs homing into the decellularized tissue (Santoro, et al., 2016). Based on their previous experience on perfusion-based cellularization of large scaffolds (Santoro, et al., 2010), and on the reported advantage of mass transport through scaffolds to generate large engineered tissues (Wendt, et al., 2005, Wendt, et al., 2003a), the inventors exploited the characteristics of a perfusion culture system to force penetration of VICs inside the pericardium matrix and observe, for the first time, their behavior. Remarkably, the present results (Figure 5) indicated a marked downregulation of the activated VICs marker αSMA only in cells that grew inside the matrix and not those covering the two surfaces. While this result is consistent with the reversibility of VICs activation process (Schroer and Merryman, 2015), it also highlights the fundamental role of these cells as possible 'mechanosensors', which may act in detection of local matrix stiffness at the crossroad between self-renewal or pathologic progression of valve tissue (Chen, et al., 2009, David Merryman, 2010, Fisher, et al., 2013, Liu, et al., 2007b, Ma, et al., 2017, Wang, et al., 2014, Yip, et al., 2009). In this respect, cells colonizing the inner pericardium layers may contribute to novel matrix deposition and to mechanical maturation of the matrix itself, and thus to establish specific cell mechanics criteria to be met in order to manufacture artificial valve tissue endowed with self-regeneration capacity (Parvin Nejad, et al., 2016, Pesce and Santoro, 2017).

In conclusion, the present invention establishes an efficient and potentially industrially scalable method to obtain a substantial growth of valve competent cells in mammal-derived pericardium with the potential to be employed in the manufacturing of living tissue for valve replacement therapies.

## BIBLIOGRAPHIC REFERENCES

Allain CC, Poon LS, Chan CSG, et al. 1974, Clinical Chemistry, 20 (4): 470.

Banfi C, Brioschi M, Barcella S, et al. 2009, PROTEOMICS, 9 (5): 1344-1352.

Boer U, Lohrenz A, Klingenberg M, et al. 2012, Biomaterials, 32 (36): 9730-9737.

Brioschi M, et al. 2014, Analytical and Bioanalytical Chemistry, 406 (12): 2817-2825.

Brioschi M, Lento S, Tremoli E, et al. 2013, Journal of proteomics, 78: 346-361.

Carpentier A, et al. 1969, The J. of thoracic and cardiovascular surgery, 58 (4): 467-483.

Chacko SJ, et al. 2013, Circulation Cardiovascular imaging, 6 (5): 776-783.

Chen JH, Yip CY, Sone ED, et al. 2009, Am J Pathol, 174 (3): 1109-1119.

Cioffi M, Kuffer J, Strobel S, et al. 2008, Journal of biomechanics, 41 (14): 2918-2925.

Converse GL, et al. 2017, J. of Biomed. Mat. Res. Part B: Applied Biomaterials, 105 (2): 249-259.

Dainese L, Guarino A, Burba I, et al. 2012, J Heart Valve Dis, 21 (1): 125-134.

David Merryman W. 2010, Journal of Biomechanics, 43 (1): 87-92.

David TE. 2013, Nat Rev Cardiol, 10 (7): 375-386.

Farias FR, et al. 2012, Interactive cardiovascular and thoracic surgery, 15 (2): 229-234.

Fisher C, Chen J, Merryman WD. 2013, Biomech Model Mechanobiol, 12 (1): 5-17.

Flameng W, et al. 2014, The J. of thoracic and cardiovascular surgery, 147 (4): 1219-1224.

Folch J, Lees M, Stanley GHS. 1957, J. of Biological Chemistry, 226 (1): 497-509.

Gardin C, Ricci S, Ferroni L, et al. 2015, PloS one, 10 (7): e0132344.

Gatto C, Giurgola L, D'Amato Tothova J. 2013, Cell and tissue banking, 14 (1): 107-115.

Ghodsizad A, Bordel V, Wiedensohler H, et al. 2014, ASAIO journal (American Society for Artificial Internal Organs : 1992), 60 (5): 582-586.

Grabenwoger M, Sider J, Fitzal F, et al. 1996, Ann Thorac Surg, 62 (3): 772-777.

Guldner NW, Jasmund I, Zimmermann Hr, et al. 2009, Circulation, 119 (12): 1653-1660.

Head SJ, Celik M, Kappetein AP. 2017, Eur Heart J. 38(28): 2183-2191.

Horke A. 2016, Eur J Cardiothorac Surg, 49 (3): 707-708.

Johnston DR, et al. 2015, The Annals of thoracic surgery, 99 (4): 1239-1247.

Korossis SA, Booth C, Wilcox HE, et al. 2002, J Heart Valve Dis, 11 (4): 463-471.

Laemmli UK. 1970, Nature, 227 (5259): 680-685.

Leong J, Munnelly A, Liberio B, et al. 2013, J. of biom. applications, 27 (8): 948-960.

Lim H-G, Kim SH, Choi SY, et al. 2012, European Journal of Cardio-Thoracic Surgery, 41 (2): 383-390.

Liu AC, Joag VR, Gotlieb AI. 2007a, The Am. J. of Pathology, 171 (5): 1407-1418.
Liu AC, Joag VR, Gotlieb AI. 2007b, The Am. J. of Pathology, 171 (5): 1407-1418.
Liu ZZ, Wong ML, Griffiths LG. 2016, Scientific reports, 6: 37089.
Ma H, Killaars AR, DelRio FW, et al. 2017, Biomaterials, 131: 131-144.
Mirsadraee S, Wilcox HE, Korossis SA, et al. 2006, Tissue Eng, 12 (4): 763-773.
Mirsadraee S, et al. 2007, The Journal of surgical research, 143 (2): 407-414.
Namiri M, et al. 2017, J. of tissue engine. and regenerative med., 11 (5): 1675-1683.
Nishimura RA, Otto CM, Bonow RO, et al. 2014, Circulation, 129 (23): e521-643.
Okamoto Y, Yamamoto K, Yoshii S. 2016, Journal of cardiac surgery, 31 (4): 195-202.
Paola A, et al. 2017, Biomedical Materials, 12 (1): 015021.
Parvin Nejad S, et al. 2016, Advanced drug delivery reviews, 96: 161-175.
Pesce M, Santoro R. 2017, Pharmacology & Therapeutics, 171: 75-82.
Porras AM, et al. 2017, Journal of the American Heart Association, 6 (3).
Roosens A, et al. 2016, Annals of biomedical engineering, 44 (9): 2827-2839.
Rossi MA, Braile DM, Teixeira MD, et al. 1990, J Exp Pathol (Oxford), 71 (2): 187-196.
Roverso M, et al. 2016, Eur. J. of mass spectrometry (Chichester, England), 22 (2): 71-82.
Santoro R, et al. 2016, J. of Biom. Mat. Res. Part B: Applied Biomat., 104 (2): 345-356.
Santoro R, et al. 2012, J. of tissue engineering and regenerative medicine, 6 (9): 696-701.
Santoro R, Olivares AL, Brans G, et al. 2010, Biomaterials, 31 (34): 8946-8952.
Schoen FJ. 2008, Circulation, 118 (18): 1864-1880.
Schoen FJ, Gotlieb AI. 2016, Cardiovascular pathology : the official journal of the Society for Cardiovascular Pathology, 25 (4): 341-352.
Schoen FJ, Levy RJ. 2005, The Annals of thoracic surgery, 79 (3): 1072-1080.
Schroer AK, Merryman WD. 2015, J Cell Sci, 128 (10): 1865-1875.
Seddon AM, et al., 2004, Biochimica et biophysica acta, 1666 (1-2): 105-117.
Siddiqui RF, Abraham JR, Butany J. 2009, Histopathology, 55 (2): 135-144.
Taylor PM, Batten P, Brand NJ, et al. 2003, Intern J Biochem Cell Biol, 35 (2): 113-118.
Vahanian A, et al. 2012, European heart journal, 33 (19): 2451-2496.
Vinci MC, Tessitore G, Castiglioni L, et al. 2013, PLoS ONE, 8 (5): e64769.
Wang H, Leinwand LA, Anseth KS. 2014, Nat Rev Cardiol, 11 (12): 715-727.
Wendt D, Jakob M, Martin I. 2005, J. of Bioscience and Bioengineering, 100 (5): 489-494.
Wendt D, et al. 2003a, Biotechnology and Bioengineering, 84 (2): 205-214.
Wendt D, et al. 2003b, Biotechnology and bioengineering, 84 (2): 205-214.
Wong ML, Wong JL, Athanasiou KA, et al. 2013, Acta Biomaterialia, 9 (5): 6492-6501.
Wong ML, Wong JL, Vapniarsky N, et al. 2016, Biomaterials, 92: 1-12.
Yap KH, et al. 2013, Interact Cardiovasc Thorac Surg, 16 (3): 361-373.
Yip CY, Chen JH, Zhao R, et al. 2009, Arterioscler Thromb Vasc Biol, 29 (6): 936-942.

**Claims**

1. An *ex vivo* method for producing decellularized pericardial material comprising:

   a) incubation of a pericardial tissue in a hypotonic solution supplemented with at least one protease inhibitor;
   b) incubation of the pericardial tissue incubated in a) in a 1% (v/v) Triton X-100 solution in bi-distilled water;
   c) incubation of the pericardial tissue incubated in b) into a 0.1% (w/v) sodium dodecylsulphate (SDS) in bi-distilled water.

2. The *ex vivo* method according to claim 1, wherein the hypotonic solution is 10 mM Tris-HCl having a pH 8.0 or wherein any of steps a), b) or c) is carried out for 1 to 24 hours at a temperature between 2 and 8 °C.

3. The *ex vivo* method according to claim 1 or 2, wherein said pericardial tissue is porcine, bovine, human, equine.

4. A decellularized pericardial material obtainable by the *ex vivo* method according to claim 1 to 3.

5. The decellularized pericardial material according to claim 4, **characterized in that** it has a permeability lower than $1.0 \times 10^{-13}$ m$^4$/Ns.

6. The decellularized pericardial material according to claim 4 or 5, **characterized in that** it has a thickness comprised between 100 μm and 1000μm.

7. The decellularized pericardial material according to any one of claims 4 to 6, **characterized in that** essentially all cellular proteins have been removed.

8. Use of the decellularized pericardial material according to any one of claim 4 to 7 for the production of living tissue incorporating cells.

9. An *ex vivo* method for producing recellularized pericardial tissue comprising contacting the decellularized pericardial material as defined in any one of claim 4 to 7 with autologous or heterologous cells selected from the group consisting of: valve-derived interstitial cells, adipose-tissue stromal/mesenchymal cells, myocardial stromal/mesenchymal cells, Wharton Jelly stromal/mesenchymal cells and vascular-derived stromal/mesenchymal/progenitor cells or a mixture thereof, by means of an oscillatory flow, preferably in a bioreactor.

10. The *ex vivo* method for producing recellularized pericardial tissue according to claim 9 wherein the oscillatory flow has a flow rate between 0.01 ml/min and 50 ml/min, preferably from 0.03 ml/min to 3 ml/min.

11. A recellularized pericardial tissue obtainable from the *ex vivo* method according to any one of claims 9 or 10.

12. Use of the recellularized pericardial tissue according to claim 11 for the production of heart valve leaflet, preferably for the manufacture of conventional and mini-invasively implantable valve prostheses or for use in surgical reconstruction of an aortic valve and/or a mitral valve.

13. An *ex vivo* method for producing a heart valve leaflet or for producing a recellularized blood vessel, said leaflet or said blood vessel comprising autologous or heterologous cells selected from the group consisting of: valve-derived interstitial cells, adipose-tissue stromal/mesenchymal cells, myocardial stromal/mesenchymal cells, Wharton Jelly stromal/mesenchymal cells and vascular-derived stromal/mesenchymal/progenitor cells or a mixture thereof, said method comprising the steps of:

   a) introducing said autologous or heterologous cells into the decellularized pericardial material as defined in any one of claim 4 to 7 by means of an oscillatory flow thereby producing a recellularized pericardial tissue;
   b) cutting and/or trimming the recellularized pericardial tissue, preferably by means of a valve leaflet sizer, preferably to form a blood vessel.

14. The heart valve leaflet or the recellularized blood vessel obtainable by the method according to claim 13.

15. The decellularized pericardial material according to claims 4 to 7, the cellular pericardial tissue according to claim 11 or the heart valve leaflet or the recellularized blood vessel according to claim 14 for use in surgery or for medical use, preferably for use in the treatment of diseased and/or damaged heart tissue, preferably said diseased and/or damaged heart tissue is a calcific aortic valve disease, aortic valve insufficiency, mitral prolapse, mitral stenosis, preferably said surgery is an implantation of a heart valve, a replacement of a heart valve, a repair of a heart valve or a heart valve limb reconstruction, preferably said heart valve leaflets reconstruction is an Ozaki procedure, a Ross Procedure, preferably for use in the treatment of vascular disease, preferably the vascular disease is a peripheral and/or coronary artery disease, preferably for use for replacement of a peripheral and/or coronary artery in peripheral and/or coronary bypass grafting.

Fig. 1

Fig. 2

Fig. 3

Fig. 4

Fig. 5

Fig. 6

Fig. 7

A: Graduated Column
B: Graduated Capillary
P: Pericardium sample
F: Filter
ΔP: Hydraulic pressure
$T_i$: Time initial
$T_f$: Time final
○ : Gasket

Fig. 8

Table 2

EP 3 533 475 A1

| Description | Accession | Peptide count | Unique peptides | Confidence score |
|---|---|---|---|---|
| Glyceraldehyde-3-phosphate dehydrogenase OS=Sus scrofa GN=GAPDH PE=2 SV=1 | A0A076KRX0 | 4 | 4 | 30,29 |
| Osteoglycin/mimecan OS=Sus scrofa GN=OGN PE=2 SV=1 | A0A0H5ANC0 | 13 | 11 | 113,06 |
| Membrane glycoprotein (Fragment) OS=Sus scrofa GN=thy-1 PE=2 SV=1 | A0A1V0CLY1 | 3 | 2 | 22,58 |
| Heat shock 70kDa protein 1A OS=Sus scrofa GN=HSPA1A PE=3 SV=1 | A5A8V6 | 2 | 2 | 19,25 |
| Beta-globin (Fragment) OS=Sus scrofa PE=2 SV=1 | A7YB43 | 6 | 6 | 51,44 |
| Beta-enolase 3 transcript variant 1 OS=Sus scrofa GN=ENO3 PE=2 SV=1 | B1A3A0 | 2 | 2 | 10,62 |
| Trypsinogen OS=Sus scrofa PE=2 SV=1 | C5IWV5 | 5 | 4 | 28,64 |
| Actin alpha 2 OS=Sus scrofa GN=ACTA2 PE=1 SV=1 | C7AI81 | 12 | 3 | 91,09 |
| Triosephosphate isomerase OS=Sus scrofa GN=TPI1 PE=1 SV=1 | D0G7F6 | 3 | 2 | 14,93 |
| Tropomyosin 4 OS=Sus scrofa GN=TPM4 PE=2 SV=1 | D0G7F7 | 5 | 4 | 36,40 |
| Protein disulfide-isomerase OS=Sus scrofa GN=grp-58 PE=1 SV=1 | E1CAJ5 | 2 | 2 | 13,06 |
| Profilin OS=Sus scrofa GN=PFN1 PE=1 SV=1 | F1RFY1 | 2 | 2 | 14,41 |
| Transforming growth factor-beta-induced protein ig-h3 OS=Sus scrofa GN=TGFBI PE=1 SV=2 | F1RHA7 | 16 | 16 | 144,71 |
| Uncharacterized protein OS=Sus scrofa GN=ACTN4 PE=1 SV=2 | F1RI39 | 3 | 2 | 17,35 |
| Uncharacterized protein OS=Sus scrofa GN=LOC100525453 PE=1 SV=2 | F1RJT3 | 2 | 2 | 13,86 |
| Neurofilament medium polypeptide OS=Sus scrofa GN=NEFM PE=1 SV=2 | F1RJU6 | 11 | 5 | 82,67 |
| Uncharacterized protein OS=Sus scrofa GN=PEBP1 PE=1 SV=1 | F1RKG8 | 2 | 2 | 12,44 |
| Prelamin-A/C OS=Sus scrofa GN=LMNA PE=1 SV=1 | F1RLQ2 | 7 | 6 | 47,48 |
| Histone H2A OS=Sus scrofa GN=LOC100154508 PE=3 SV=1 | F1RPK0 | 3 | 3 | 26,40 |
| Dermatopontin OS=Sus scrofa GN=DPT PE=4 SV=1 | F1RPV5 | 4 | 3 | 28,29 |
| Uncharacterized protein OS=Sus scrofa PE=1 SV=2 | F1RQI0 | 26 | 24 | 195,64 |
| Uncharacterized protein OS=Sus scrofa PE=1 SV=2 | F1RQI2 | 3 | 3 | 25,61 |
| Uncharacterized protein OS=Sus scrofa PE=4 SV=2 | F1RT61 | 5 | 2 | 29,16 |
| Serum albumin OS=Sus scrofa GN=ALB PE=1 SV=1 | F1RUN2 | 5 | 4 | 34,27 |
| Histone H3 OS=Sus scrofa PE=3 SV=1 | F1RVA0 | 6 | 6 | 37,89 |
| Uncharacterized protein OS=Sus scrofa GN=COL3A1 PE=1 SV=2 | F1RYI8 | 4 | 3 | 24,32 |
| Uncharacterized protein OS=Sus scrofa PE=1 SV=2 | F1RZU6 | 5 | 5 | 29,53 |

Fig. 9 (1/3)

# Table 2

| Description | Accession | | | |
|---|---|---|---|---|
| Uncharacterized protein OS=Sus scrofa GN=LOC100522225 PE=4 SV=2 | F1S023 | 5 | 3 | 29,67 |
| Annexin OS=Sus scrofa GN=ANXA2 PE=1 SV=2 | F1S073 | 17 | 17 | 131,21 |
| Uncharacterized protein OS=Sus scrofa GN=COL14A1 PE=1 SV=2 | F1S285 | 33 | 31 | 227,36 |
| Uncharacterized protein OS=Sus scrofa GN=PRELP PE=1 SV=2 | F1S6B4 | 15 | 15 | 133,98 |
| Fibromodulin OS=Sus scrofa GN=FMOD PE=1 SV=2 | F1S6B5 | 11 | 11 | 121,07 |
| Uncharacterized protein OS=Sus scrofa GN=FBLN5 PE=1 SV=2 | F1SD87 | 4 | 3 | 25,59 |
| Uncharacterized protein OS=Sus scrofa GN=COL1A2 PE=1 SV=1 | F1SFA7 | 23 | 21 | 132,18 |
| Uncharacterized protein OS=Sus scrofa GN=KRT8 PE=1 SV=1 | F1SGG2 | 5 | 2 | 31,62 |
| Uncharacterized protein OS=Sus scrofa GN=PRPH PE=1 SV=2 | F1SHC0 | 8 | 4 | 47,92 |
| Tubulin alpha chain OS=Sus scrofa GN=TUBA1C PE=1 SV=1 | F1SHC1 | 9 | 9 | 66,76 |
| Annexin OS=Sus scrofa GN=ANXA1 PE=1 SV=2 | F1SJB5 | 2 | 2 | 12,45 |
| Transgelin OS=Sus scrofa GN=TAGLN PE=1 SV=2 | F1SJS8 | 2 | 2 | 15,62 |
| Galectin OS=Sus scrofa GN=LGALS1 PE=1 SV=1 | F1SKM9 | 6 | 5 | 47,93 |
| Uncharacterized protein OS=Sus scrofa PE=1 SV=2 | F1SL03 | 2 | 2 | 12,04 |
| Uncharacterized protein OS=Sus scrofa PE=1 SV=1 | F1SMI5 | 34 | 29 | 264,81 |
| Protein AMBP OS=Sus scrofa GN=AMBP PE=1 SV=1 | F1SN71 | 2 | 2 | 10,18 |
| Uncharacterized protein OS=Sus scrofa GN=KERA PE=1 SV=1 | F1SQ08 | 5 | 5 | 37,39 |
| Uncharacterized protein OS=Sus scrofa GN=LUM PE=1 SV=1 | F1SQ09 | 11 | 10 | 112,33 |
| Decorin OS=Sus scrofa GN=DCN PE=4 SV=1 | F1SQ10 | 20 | 19 | 221,95 |
| Endoplasmin OS=Sus scrofa GN=HSP90B1 PE=1 SV=2 | F1SRK6 | 3 | 3 | 24,13 |
| Uncharacterized protein OS=Sus scrofa GN=MYH10 PE=1 SV=2 | F1SSA6 | 8 | 5 | 42,12 |
| Uncharacterized protein OS=Sus scrofa GN=ASPN PE=1 SV=1 | F1SUE4 | 13 | 13 | 110,49 |
| Uncharacterized protein OS=Sus scrofa PE=1 SV=1 | F1SUM3 | 2 | 2 | 14,66 |
| Peptidyl-prolyl cis-trans isomerase OS=Sus scrofa GN=ppia PE=2 SV=1 | F2Q9A3 | 2 | 2 | 15,19 |
| Histone H2B OS=Sus scrofa GN=HIST1H2BD PE=3 SV=1 | F2Z584 | 8 | 8 | 58,06 |
| Annexin OS=Sus scrofa GN=ANXA5 PE=1 SV=2 | F2Z5C1 | 8 | 8 | 65,38 |
| Uncharacterized protein OS=Sus scrofa GN=AHNAK PE=1 SV=1 | I3L5D5 | 9 | 7 | 58,33 |
| Uncharacterized protein OS=Sus scrofa PE=1 SV=1 | I3LC83 | 16 | 7 | 106,91 |
| Uncharacterized protein OS=Sus scrofa PE=1 SV=1 | I3LG96 | 3 | 3 | 16,76 |
| Uncharacterized protein OS=Sus scrofa GN=DPYSL2 PE=1 SV=1 | I3LJE2 | 2 | 2 | 15,49 |
| Uncharacterized protein OS=Sus scrofa PE=1 SV=1 | I3LIX2 | 5 | 2 | 30,36 |

Fig. 9 (2/3)

# Table 2

| Protein | Accession | | | |
|---|---|---|---|---|
| Uncharacterized protein OS=Sus scrofa GN=COL6A2 PE=1 SV=1 | I3LQ84 | 32 | 27 | 335,09 |
| Uncharacterized protein OS=Sus scrofa PE=1 SV=1 | I3LS72 | 16 | 10 | 130,93 |
| Uncharacterized protein OS=Sus scrofa PE=4 SV=1 | I3LUM2 | 5 | 2 | 28,99 |
| Uncharacterized protein OS=Sus scrofa GN=ACTG1 PE=1 SV=1 | I3LVD5 | 16 | 8 | 166,34 |
| Biglycan OS=Sus scrofa GN=BGN PE=1 SV=1 | K7GPS5 | 15 | 14 | 144,08 |
| Uncharacterized protein (Fragment) OS=Sus scrofa PE=1 SV=1 | K7GRK7 | 19 | 9 | 122,03 |
| Elongation factor 1-alpha OS=Sus scrofa GN=EEF1A PE=1 SV=1 | Q0PY11 | 4 | 4 | 28,91 |
| Type VI collagen alpha-3 chain (Fragment) OS=Sus scrofa GN=COL6A3 PE=2 SV=1 | Q1T7A4 | 7 | 6 | 73,71 |
| Type VI collagen alpha-3 chain (Fragment) OS=Sus scrofa GN=COL6A3 PE=2 SV=1 | Q1T7A5 | 24 | 24 | 229,88 |
| Type VI collagen alpha-1 chain (Fragment) OS=Sus scrofa GN=COL6A1 PE=2 SV=1 | Q1T7A8 | 4 | 4 | 25,25 |
| Type VI collagen alpha-1 chain (Fragment) OS=Sus scrofa GN=COL6A1 PE=2 SV=1 | Q1T7A9 | 8 | 6 | 63,93 |
| Nebulin-related anchoring protein OS=Sus scrofa GN=NRAP PE=2 SV=2 | Q3Y5G4 | 2 | 2 | 17,94 |
| Histone H1.2-like protein OS=Sus scrofa PE=3 SV=1 | Q4TTS4 | 2 | 2 | 13,06 |
| Procollagen alpha 2(V) OS=Sus scrofa GN=COL5A2 PE=2 SV=1 | Q59IP2 | 20 | 19 | 112,05 |

Fig. 9 (3/3)

Europäisches
Patentamt

European
Patent Office

Office européen
des brevets

# EUROPEAN SEARCH REPORT

Application Number

EP 18 15 9149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/087428 A1 (WOLFINBARGER LLOYD [US] ET AL) 8 May 2003 (2003-05-08) | 1-8,15 | INV. A61L27/36 A61L27/38 |
| Y | * 6, 24-38, 49, 53, 58-59; claims 38-50, 56-69 * | 9-14 | |
| A | SAEED MIRSADRAEE ET AL: "Development and Characterization of an Acellular Human Pericardial Matrix for Tissue Engineering", TISSUE ENGINEERING, vol. 12, no. 4, 1 April 2006 (2006-04-01), pages 763-773, XP055501807, US ISSN: 1076-3279, DOI: 10.1089/ten.2006.12.763 * Abstract, p. 764, "decellularization" * | 1-15 | |
| Y | OTT H C ET AL: "Perfusion-decellularized matrix: using nature's platform to engineer a bioartificial heart", NATURE MEDICINE, NATURE PUB. CO, NEW YORK, vol. 14, no. 2, 1 February 2008 (2008-02-01), pages 213-221, XP002612651, ISSN: 1078-8956, DOI: 10.1038/NM1684 [retrieved on 2008-01-13] * Abstract, p. 216, "Whole-heart experiments" * | 9-14 | |

TECHNICAL FIELDS
SEARCHED (IPC)

A61L

The present search report has been drawn up for all claims

| Place of search | Date of completion of the search | Examiner |
|---|---|---|
| The Hague | 23 August 2018 | Cadamuro, Sergio |

EPO FORM 1503 03.82 (P04C01)

## ANNEX TO THE EUROPEAN SEARCH REPORT
## ON EUROPEAN PATENT APPLICATION NO.

EP 18 15 9149

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2018

| Patent document cited in search report | Publication date | Patent family member(s) | Publication date |
|---|---|---|---|
| US 2003087428 A1 | 08-05-2003 | US 2003087428 A1<br>US 2004076657 A1<br>US 2009041729 A1<br>US 2011143438 A1<br>US 2014065238 A1 | 08-05-2003<br>22-04-2004<br>12-02-2009<br>16-06-2011<br>06-03-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALLAIN CC ; POON LS ; CHAN CSG et al.** *Clinical Chemistry,* 1974, vol. 20 (4), 470
- **BANFI C ; BRIOSCHI M ; BARCELLA S et al.** *PROTEOMICS,* 2009, vol. 9 (5), 1344-1352
- **BOER U ; LOHRENZ A ; KLINGENBERG M et al.** *Biomaterials,* 2012, vol. 32 (36), 9730-9737
- **BRIOSCHI M et al.** *Analytical and Bioanalytical Chemistry,* 2014, vol. 406 (12), 2817-2825
- **BRIOSCHI M ; LENTO S ; TREMOLI E et al.** *Journal of proteomics,* 2013, vol. 78, 346-361
- **CARPENTIER A et al.** *The J. of thoracic and cardiovascular surgery,* 1969, vol. 58 (4), 467-483
- **CHACKO SJ et al.** *Circulation Cardiovascular imaging,* 2013, vol. 6 (5), 776-783
- **CHEN JH ; YIP CY ; SONE ED et al.** *Am J Pathol,* 2009, vol. 174 (3), 1109-1119
- **CIOFFI M ; KUFFER J ; STROBEL S et al.** *Journal of biomechanics,* 2008, vol. 41 (14), 2918-2925
- **CONVERSE GL et al.** Applied Biomaterials. *J. of Biomed. Mat. Res.,* 2017, vol. 105 (2), 249-259
- **DAINESE L ; GUARINO A ; BURBA I et al.** *J Heart Valve Dis,* 2012, vol. 21 (1), 125-134
- **DAVID MERRYMAN W.** *Journal of Biomechanics,* 2010, vol. 43 (1), 87-92
- **DAVID TE.** *Nat Rev Cardiol,* 2013, vol. 10 (7), 375-386
- **FARIAS FR et al.** *Interactive cardiovascular and thoracic surgery,* 2012, vol. 15 (2), 229-234
- **FISHER C ; CHEN J ; MERRYMAN WD.** *Biomech Model Mechanobiol,* 2013, vol. 12 (1), 5-17
- **FLAMENG W et al.** *The J. of thoracic and cardiovascular surgery,* 2014, vol. 147 (4), 1219-1224
- **FOLCH J ; LEES M ; STANLEY GHS.** *J. of Biological Chemistry,* 1957, vol. 226 (1), 497-509
- **GARDIN C ; RICCI S ; FERRONI L et al.** *PloS one,* 2015, vol. 10 (7), e0132344
- **GATTO C ; GIURGOLA L ; D'AMATO TOTHOVA J.** *Cell and tissue banking,* 2013, vol. 14 (1), 107-115
- **GHODSIZAD A ; BORDEL V ; WIEDENSOHLER H et al.** *ASAIO journal (American Society for Artificial Internal Organs : 1992),* 2014, vol. 60 (5), 582-586
- **GRABENWOGER M ; SIDER J ; FITZAL F et al.** *Ann Thorac Surg,* 1996, vol. 62 (3), 772-777
- **GULDNER NW ; JASMUND I ; ZIMMERMANN HR et al.** *Circulation,* 2009, vol. 119 (12), 1653-1660

- **HEAD SJ ; CELIK M ; KAPPETEIN AP.** *Eur Heart J.,* 2017, vol. 38 (28), 2183-2191
- **HORKE A.** *Eur J Cardiothorac Surg,* 2016, vol. 49 (3), 707-708
- **JOHNSTON DR et al.** *The Annals of thoracic surgery,* 2015, vol. 99 (4), 1239-1247
- **KOROSSIS SA ; BOOTH C ; WILCOX HE et al.** *J Heart Valve Dis,* 2002, vol. 11 (4), 463-471
- **LAEMMLI UK.** *Nature,* 1970, vol. 227 (5259), 680-685
- **LEONG J ; MUNNELLY A ; LIBERIO B et al.** *J. of biom. applications,* 2013, vol. 27 (8), 948-960
- **LIM H-G ; KIM SH ; CHOI SY et al.** *European Journal of Cardio-Thoracic Surgery,* 2012, vol. 41 (2), 383-390
- **LIU AC ; JOAG VR ; GOTLIEB AI.** *The Am. J. of Pathology,* 2007, vol. 171 (5), 1407-1418
- **LIU ZZ ; WONG ML ; GRIFFITHS LG.** *Scientific reports,* 2016, vol. 6, 37089
- **MA H ; KILLAARS AR ; DELRIO FW et al.** *Biomaterials,* 2017, vol. 131, 131-144
- **MIRSADRAEE S ; WILCOX HE ; KOROSSIS SA et al.** *Tissue Eng,* 2006, vol. 12 (4), 763-773
- **MIRSADRAEE S et al.** *The Journal of surgical research,* 2007, vol. 143 (2), 407-414
- **NAMIRI M et al.** *J. of tissue engine. and regenerative med.,* 2017, vol. 11 (5), 1675-1683
- **NISHIMURA RA ; OTTO CM ; BONOW RO et al.** *Circulation,* 2014, vol. 129 (23), e521-643
- **OKAMOTO Y ; YAMAMOTO K ; YOSHII S.** *Journal of cardiac surgery,* 2016, vol. 31 (4), 195-202
- **PAOLA A et al.** *Biomedical Materials,* 2017, vol. 12 (1), 015021
- **PARVIN NEJAD S et al.** *Advanced drug delivery reviews,* 2016, vol. 96, 161-175
- **PESCE M ; SANTORO R.** *Pharmacology & Therapeutics,* 2017, vol. 171, 75-82
- **PORRAS AM et al.** *Journal of the American Heart Association,* 2017, vol. 6 (3
- **ROOSENS A et al.** *Annals of biomedical engineering,* 2016, vol. 44 (9), 2827-2839
- **ROSSI MA ; BRAILE DM ; TEIXEIRA MD et al.** *J Exp Pathol,* 1990, vol. 71 (2), 187-196
- **ROVERSO M et al.** *Eur. J. of mass spectrometry,* 2016, vol. 22 (2), 71-82
- **SANTORO R et al.** Applied Biomat. *J. of Biom. Mat. Res.,* 2016, vol. 104 (2), 345-356
- **SANTORO R et al.** *J. of tissue engineering and regenerative medicine,* 2012, vol. 6 (9), 696-701

- **SANTORO R ; OLIVARES AL ; BRANS G et al.** *Biomaterials,* 2010, vol. 31 (34), 8946-8952
- **SCHOEN FJ.** *Circulation,* 2008, vol. 118 (18), 1864-1880
- **SCHOEN FJ ; GOTLIEB AI.** *Cardiovascular pathology : the official journal of the Society for Cardiovascular Pathology,* 2016, vol. 25 (4), 341-352
- **SCHOEN FJ ; LEVY RJ.** *The Annals of thoracic surgery,* 2005, vol. 79 (3), 1072-1080
- **SCHROER AK ; MERRYMAN WD.** *J Cell Sci,* 2015, vol. 128 (10), 1865-1875
- **SEDDON AM et al.** *Biochimica et biophysica acta,* 2004, vol. 1666 (1-2), 105-117
- **SIDDIQUI RF ; ABRAHAM JR ; BUTANY J.** *Histopathology,* 2009, vol. 55 (2), 135-144
- **TAYLOR PM ; BATTEN P ; BRAND NJ et al.** *Intern J Biochem Cell Biol,* 2003, vol. 35 (2), 113-118
- **VAHANIAN A et al.** *European heart journal,* 2012, vol. 33 (19), 2451-2496
- **VINCI MC ; TESSITORE G ; CASTIGLIONI L et al.** *PLoS ONE,* 2013, vol. 8 (5), e64769
- **WANG H ; LEINWAND LA ; ANSETH KS.** *Nat Rev Cardiol,* 2014, vol. 11 (12), 715-727
- **WENDT D ; JAKOB M ; MARTIN I.** *J. of Bioscience and Bioengineering,* 2005, vol. 100 (5), 489-494
- **WENDT D et al.** *Biotechnology and Bioengineering,* 2003, vol. 84 (2), 205-214
- **WENDT D et al.** *Biotechnology and bioengineering,* 2003, vol. 84 (2), 205-214
- **WONG ML ; WONG JL ; ATHANASIOU KA et al.** *Acta Biomaterialia,* 2013, vol. 9 (5), 6492-6501
- **WONG ML ; WONG JL ; VAPNIARSKY N et al.** *Biomaterials,* 2016, vol. 92, 1-12
- **YAP KH et al.** *Interact Cardiovasc Thorac Surg,* 2013, vol. 16 (3), 361-373
- **YIP CY ; CHEN JH ; ZHAO R et al.** *Arterioscler Thromb Vasc Biol,* 2009, vol. 29 (6), 936-942